# EUROPEAN PATENT SPECIFICATION

(11) **EP 3 978 617 B1**
(45) Date of publication and mention of the grant of the patent: **26.07.2023**
(21) Application number: 20461564.5
(22) Date of filing: 30.09.2020
(51) Int. Cl.: C12Q 1/04, G01N 33/92

(54) **A METHOD FOR DETECTING PRESENCE OF MYCOBACTERIA SPECIES IN A TEST SAMPLE**
VERFAHREN ZUM NACHWEIS DES VORHANDENSEINS VON MYCOBAKTERIEN IN EINER TESTPROBE
PROCÉDÉ PERMETTANT DE DÉTECTER LA PRÉSENCE D'ESPÈCES DE MYCOBACTÉRIES DANS UN ÉCHANTILLON D'ESSAI

(43) Date of publication of application: 06.04.2022
(73) Proprietor: Centrum Medycyny Klinicznej Dimedical Sp. z o.o., 90-702 Lodz (PL)
(72) Inventor: SZEWCZYK, Rafal, 90-702 Lodz (PL); DRUSZCZYNSKA, Magdalena, 90-702 Lodz (PL); MAJEWSKI, Karol, 90-702 Lodz (PL); KOWALSKI, Konrad, 90-702 Lodz (PL)
(74) Representative: Kancelaria Eupatent.pl Sp. z.o.o

(56) References cited:
- SZEWCZYK RAFAL ET AL: "Rapid method forMycobacterium tuberculosisidentification using electrospray ionization tandem mass spectrometry analysis of mycolic acids", DIAGNOSTIC MICROBIOLOGY AND INFECTIOUS DISEASE, ELSEVIER, AMSTERDAM, NL, vol. 76, no. 3, 11 May 2013 (2013-05-11), pages 298-305, XP028570317, ISSN: 0732-8893, DOI: 10.1016/J.DIAGMICROBIO.2013.03.025
- SONG S H ET AL: "Electrospray ionization-tandem mass spectrometry analysis of the mycolic acid profiles for the identification of common clinical isolates of mycobacterial species", JOURNAL OF MICROBIOLOGICAL METHODS, ELSEVIER, AMSTERDAM, NL, vol. 77, no. 2, 1 May 2009 (2009-05-01), pages 165-177, XP026048397, ISSN: 0167-7012, DOI: 10.1016/J.MIMET.2009.01.023 [retrieved on 2009-02-07]
- HSU F F ET AL: "Characterization of mycolic acids from the pathogen Rhodococcus equi by tandem mass spectrometry with electrospray ionization", ANALYTICAL BIOCHEMISTRY, ACADEMIC PRESS, AMSTERDAM, NL, vol. 409, no. 1, 1 February 2011 (2011-02-01), pages 112-122, XP027549216, ISSN: 0003-2697 [retrieved on 2010-10-12]

## Description

### TECHNICAL FIELD

The present invention relates to an ultrasensitive method for detecting presence of mycobacteria in a test sample, those belonging to *M*. *tuberculosis* complex (MTBc) as well as those not belonging to MTBc, called also non-tuberculous (NTM) or mycobacteria other tha tuberculosis (MOTT). The method further enables identification of the detected mycobacteria.

### BACKGROUND

Both tuberculosis (MTBc) and non-tuberculosis (NTM) species are demonstrated to cause lung diseases in humans. Among them, tuberculosis (TB) are mainly transmitted through inhalation of aerosol droplets containing MTBc, generated by patients with symptomatic disease, whereas NTM disease is mostly disseminated through aerosols originated from the environment. However, following inhalation, both Mtb and NTM are phagocytosed by alveolar macrophages in the lungs. Subsequently, various immune cells are recruited from the circulation to the site of infection.

The detection and recognition of mycobacteria species, especially at the early stage of the infection, is crucial for the outcome of treatment. Early diagnosis enables medical practitioners to start treatment as early as possible.

However, in specimens, the differential diagnosis of MTBc and NTM species is a significant challenge. Rafa 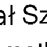 Szewczyk et al. (Diagn Microbiol Infect Dis. 2013 Jul; 76(3):298-305) discloses a rapid method for Mycobacterium tuberculosis identification using electrospray ionization tandem mass spectrometry analysis of mycolic acids.

Therefore, the present invention aims to develop the method for detecting mycobacteria in a test sample, those belonging to MTBc as well as those not belonging to NTM, and to further identify the detected mycobacteria species.

### SUMMARY

The object of the invention is a method for detecting a presence of *mycobacterium* species in a test sample according to the appended claims.

### BRIEF DESCRIPTION OF THE DRAWINGS

These and other objects of the invention presented herein are accomplished by providing a method for detecting mycobacteria in a sample. Further details and features of the present invention, its nature and various advantages will become more apparent from the following detailed description of the preferred embodiments shown in a drawing, in which:
Fig. 1 A graphic representation of the intensities of the peaks of MS/MS spectra constituting profiles of mycolic acids (MAs) obtained for the basic set of MRM pairs and the supplementary set of MRM pairs;
Fig. 2 is a schematic representation of a sample volume in the LC capillary;
Fig. 3 present a table with exemplary scanning parameters of LC-MS/MS analysis according to the present invention.

### DETAILED DESCRIPTION

The method for detecting mycobacteria as presented herein can be carried out on a sample taken from the object of interest, i.e. a human or an animal body, preferably taken from an individual suspected of being infected with mycobacteria. The collection of the sample does not form part of the invention. The test sample is preferably prepared by treating the body fluid, for example, saline/sputum, urine, blood, or a mixture thereof to extract the mycolic acids, if present in the sample, to provide a sample form that is acceptable by LC-MS/MS analysis.

The method for detecting mycobacteria involves subjecting the test sample to liquid chromatographic-tandem mass spectrometry (LC-MS/MS) analysis. The method enables detecting at least one mycobacteria species selected from the group consisting of: *M*. *tuberculosis, M. tuberculosis h37rv, M. microti, M. canetti, M. bovis caprae, M. bovis, M. africanum, M. fortuitum acetamydolyticum, M. fortuitum, M. fortuitum margeritense, M. fortuitum termophilum, M. avium, M. avium paratuberculosis, M. avium silvaticum, M. intracellulare, M. chimaera, M. scrofulaceum, M. xenopi, M. malmonese, M. simiae, M. kansasii, M. shulgai, M. gordonae, M. haemophilum, M. terrae, M. shimodei, M. chelonae, M. smegmatis, M*. *abscessus, M*. *abscessus subsp. massilense, M. marinum, M. interjectum.*

The method according to the invention is specific: it is possible to assess whether any bacteria species from the group listed above is present in the investigated sample and to identify the bacteria species. Also, the method is ultrasensitive - the presence of about 50 individual bacteria cells in the test sample is sufficient to obtain a reliable positive result.

The method according to the invention utilizes a set of reference profiles of mycolic acids (MAs), obtained for reference samples of mycobacteria species selected from the group as described above, using LC-MS/MS analysis working in MRM (multiple reaction monitoring) mode.

According to the invention, the reference profiles of MAs are compared with the obtained sample profiles of MAs, whereas based on the assessed compatibility of the profiles, there are determined: whether mycobacteria species are present in the sample, and if present - which specific species from the group listed above, is present in the investigated sample.

The reference profiles of MAs as wells as the sample profiles of MAs are obtained in the same manner by using LC-MS/MS analysis working in MRM mode, with the analyzer comprising triple quadrupole (Q1, q, Q3) instrument equipped with electrospray ion source (ESI) capable of the efficient ionization of the sample material coming from the LC instrument. In MRM, a precursor ion of interest is selected in a first mass analyzer (Q1) of the tandem mass spectrometer and fragmented in the collision cell (q), and a characteristic product ion, called fragment ion, is then selected in the second mass analyzer (Q3). Mycolic acids are thus selectively monitored using the transition pairs of ions of quadrupoles Q1 and Q3. Due to the origin, each transition pair of ions is also called an MRM pair. The first ion from the MRM pair is to be selected in the first mass analyzer (Q1) whereas the second ion from the MRM pair is to be selected in the second mass analyzer (Q3).

According to the present invention, there is disclosed a basic set of MRM pairs (Q1/Q3) including 18 MRM pairs providing a required specificity and sensitivity of the method. The set of 18 MRM pairs is dedicated to detecting the presence at least one of the mycobacteria species from the above list. The basic set of MRM pairs (Q1/Q3), denoted by m/z (mass-to-charge) ratios is set out in Table 1, column 1, below.

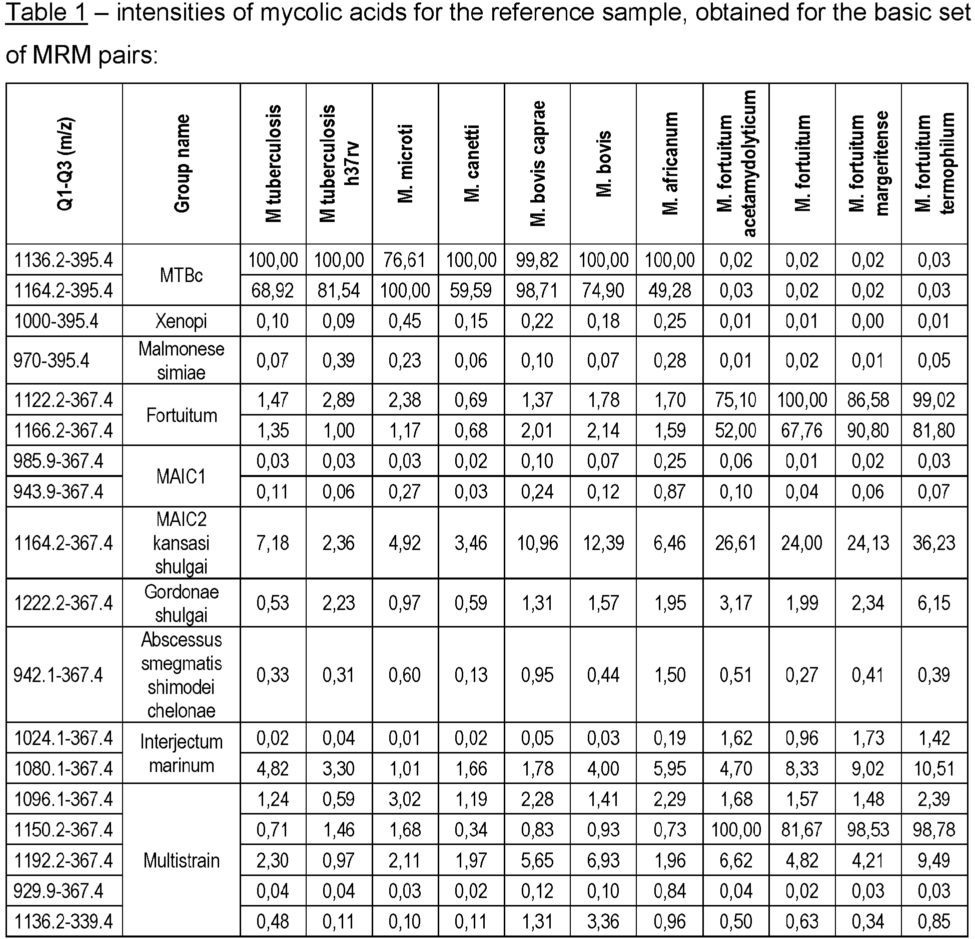

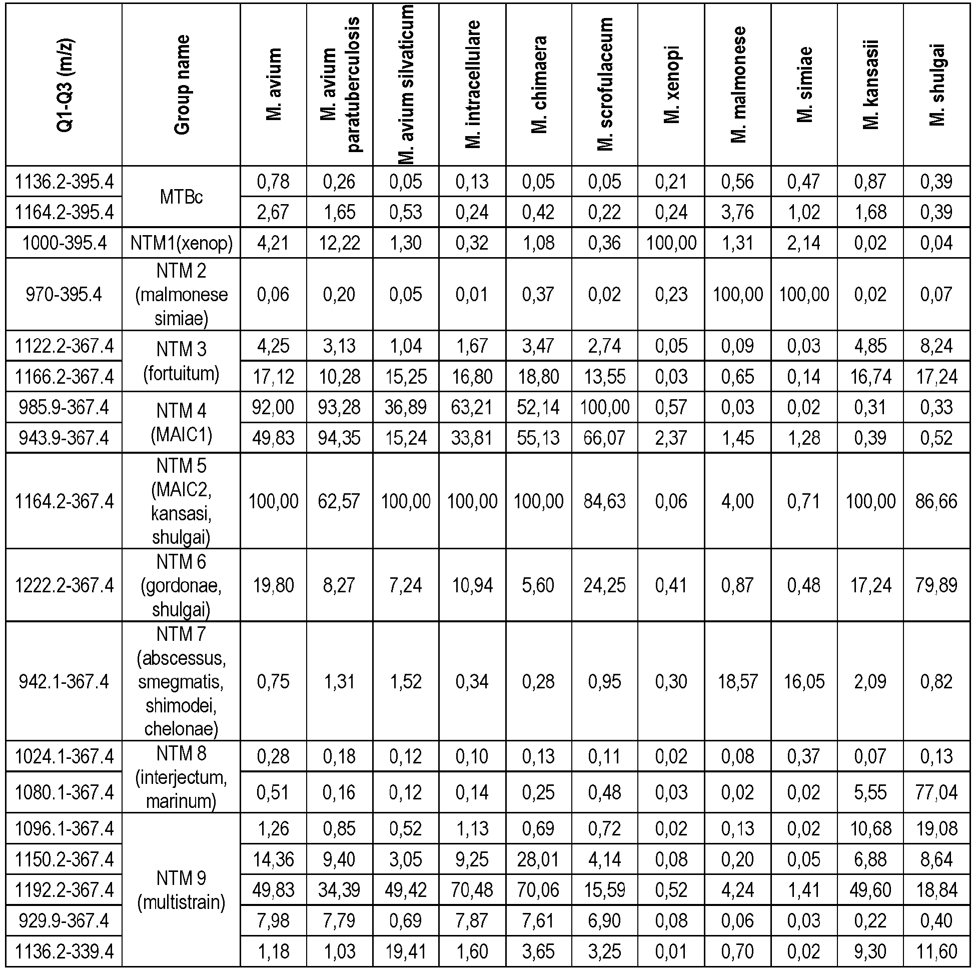

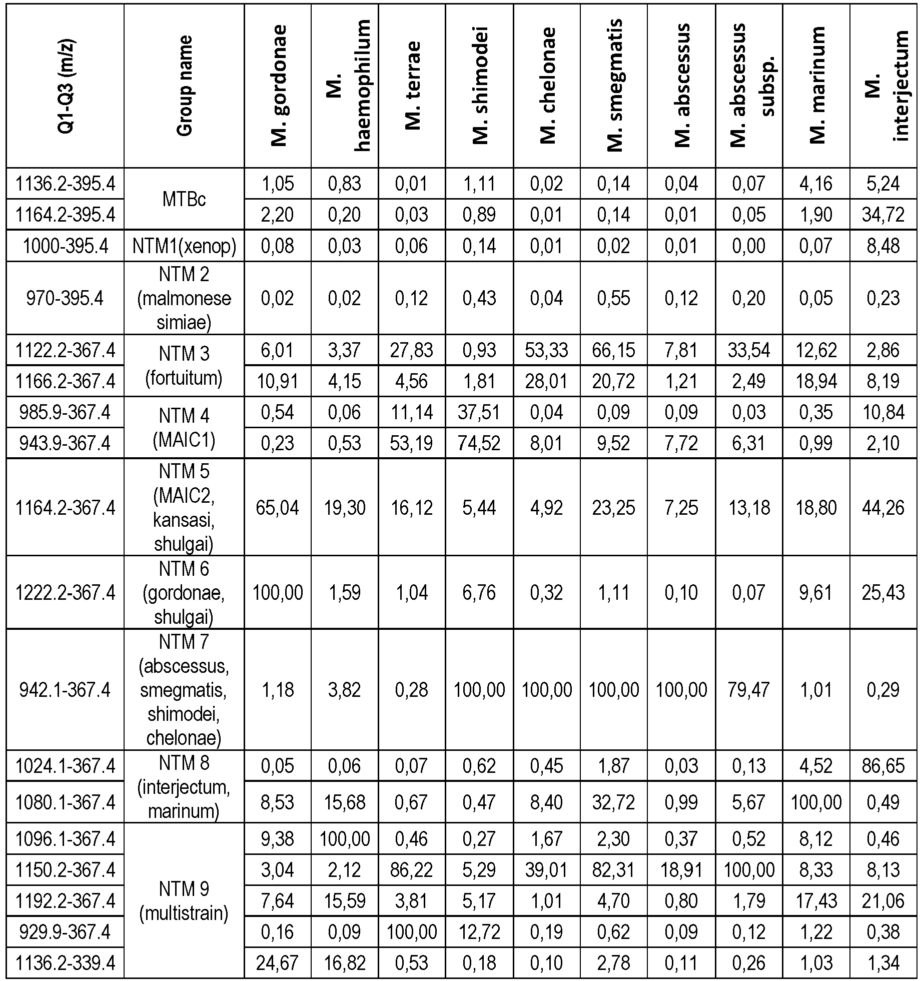

Each MRM pair listed in Table 1 enables obtaining one profile of mycolic acid (MA). This profile can be obtained (generated), only when this mycolic acid is present in the sample, thus, confirming the presence of mycobacteria in the investigated sample. In the case that the given mycobacteria species (Table 1, column 2) is present in the sample (in the required quantity - at least 50 bacterial cells), a profile of the mycolic acid is obtained, in response to the imposed selectivity, expressed by the values of MRM pair (column 1). Each profile is MS/MS spectrum (histogram), with the peaks intensities listed in Table 1, columns 3 - 13 with reference to the mycobacteria species, on a genus level..

The improved sensitivity and reliability of the method are due to the selected MRM pairs, which when applied as the analysis criteria, provide ultrasensitive detection and recognition of the mycobacteria species form the group listed above. The method is fast and easy, and it can be accomplished in the laboratory equipped with LC-MS/MS analyzer.

The selectivity and sensitivity of the method according to the invention may be further improved, by providing a set of supplementary 34 MRM pairs - applied as further criteria of LC-MS/MS analysis of reference and test sample material. The set of supplementary 34 MRM pairs with the corresponding profiles of MAs is set out in Table 2.

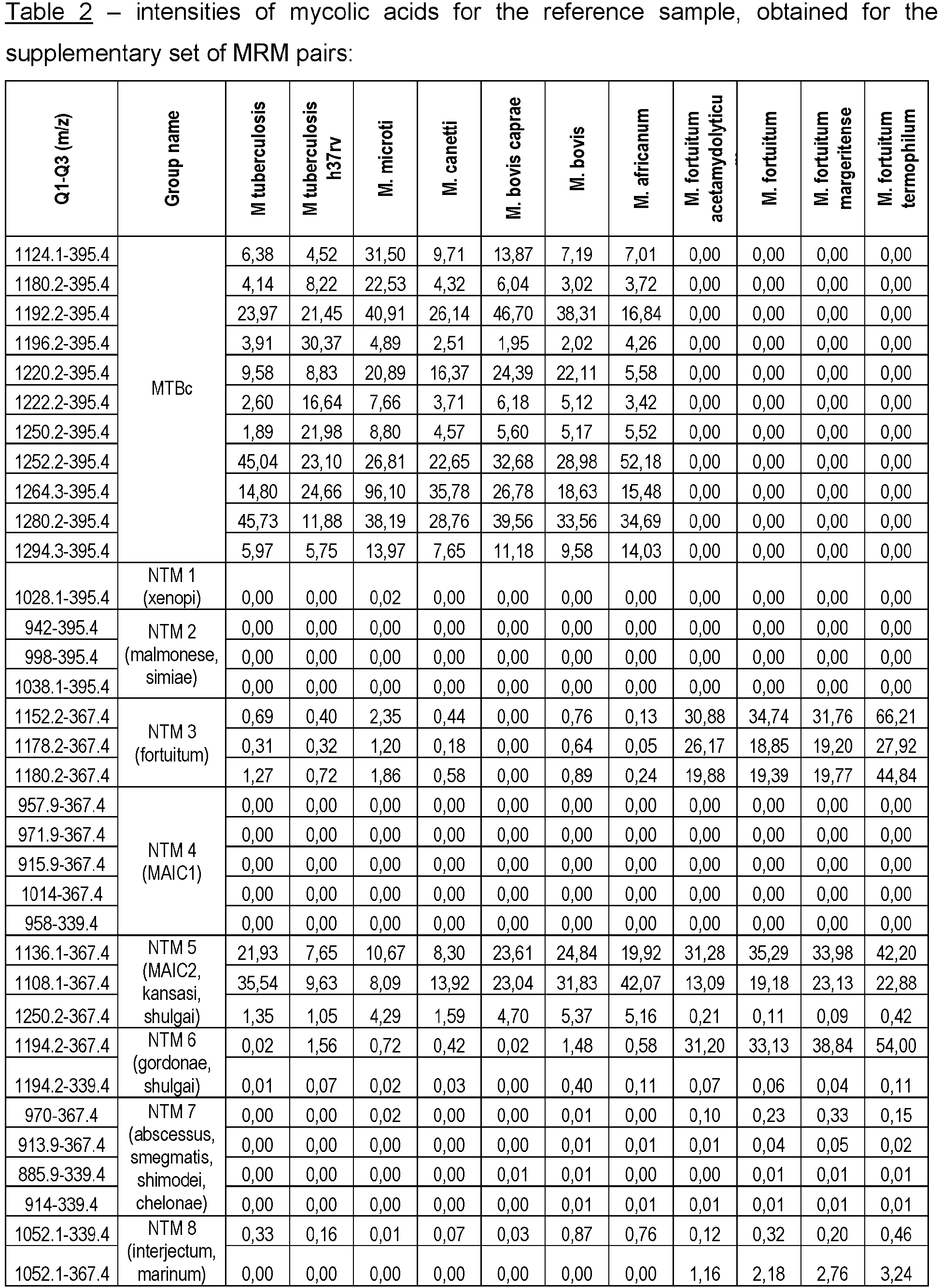

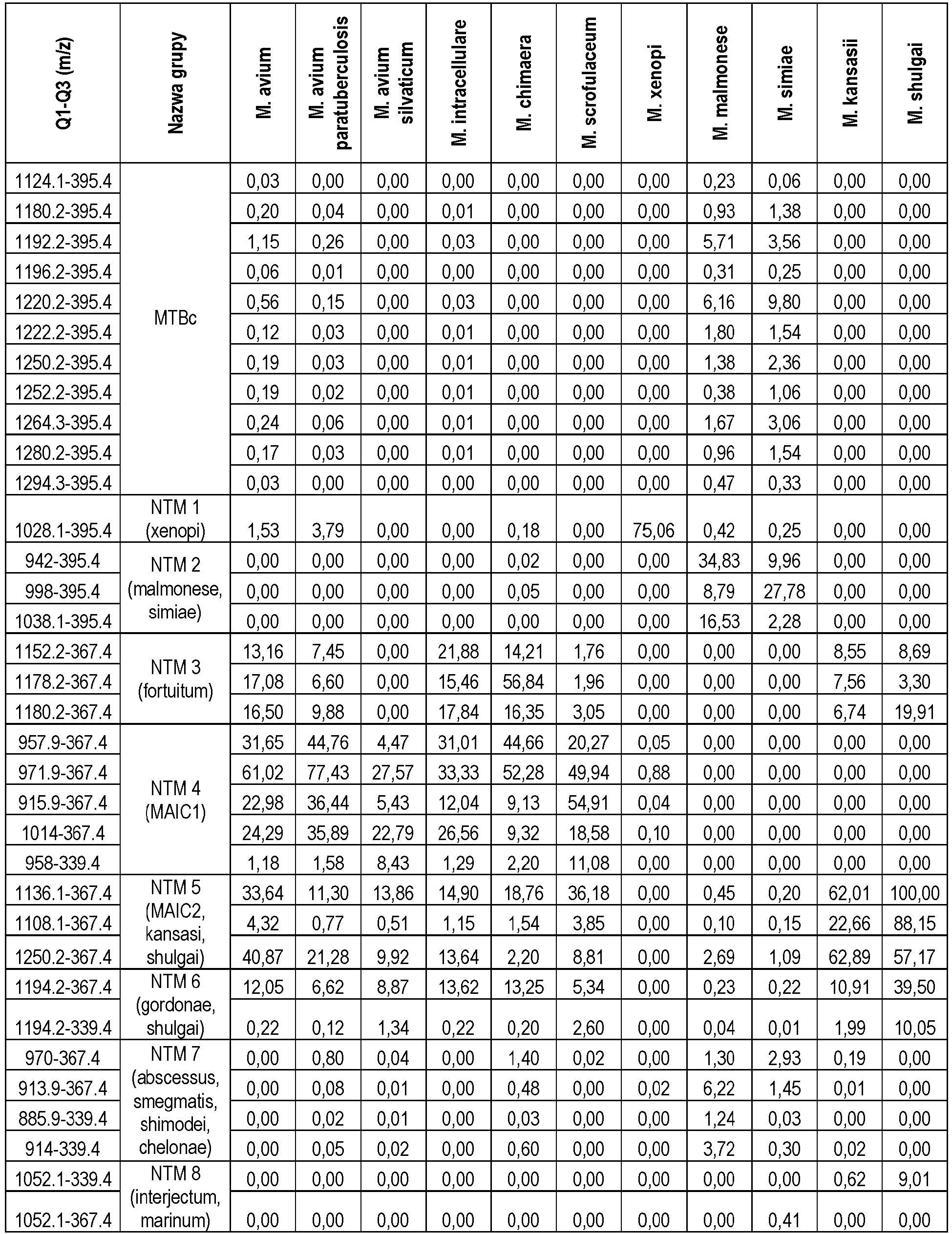

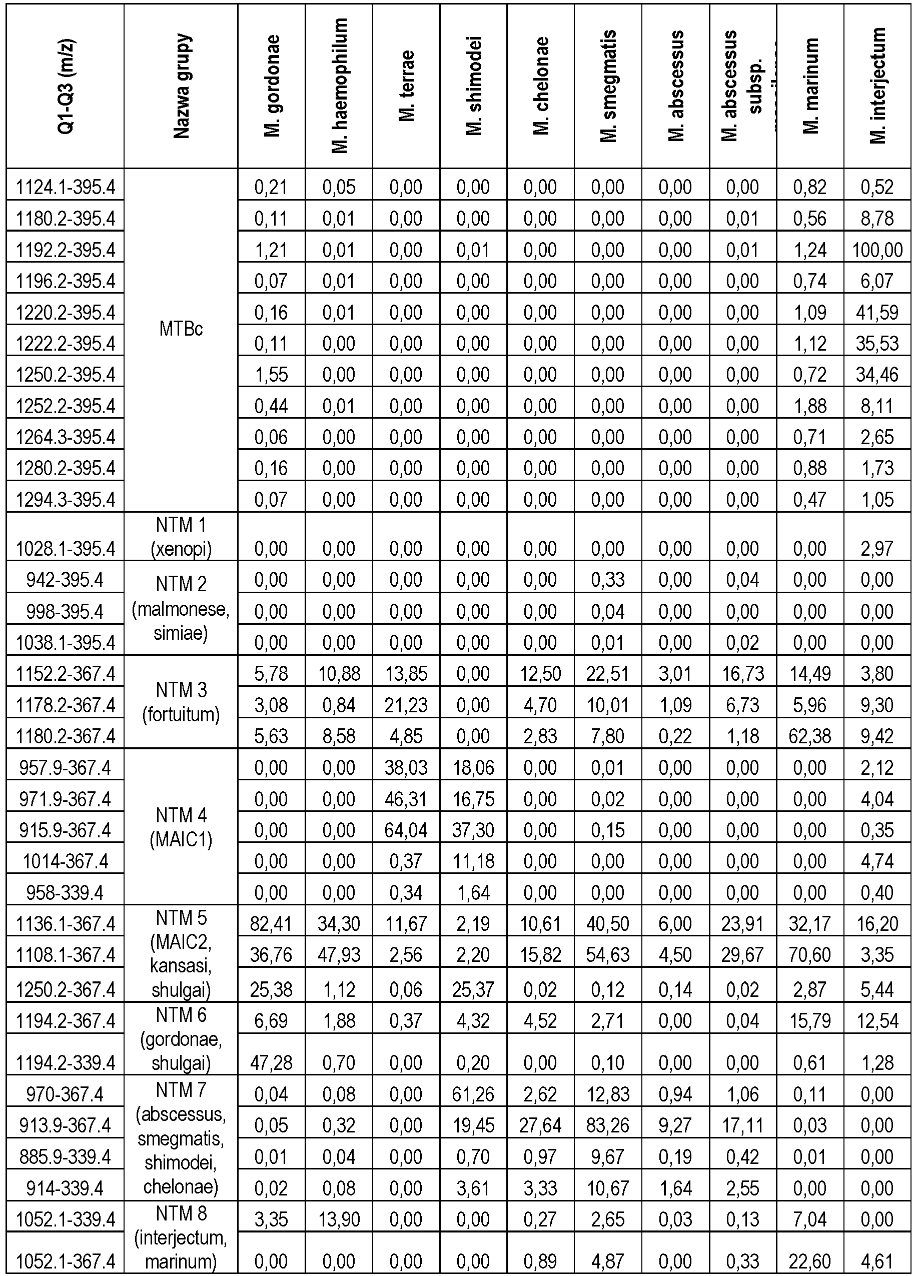

Therefore, according to the present invention, each obtained profile of MA corresponds to one MRM pair - applied as the LC-MS/MS analysis criteria. If the obtained sample profiles of MAs are compatible with the corresponding reference profiles of MAs, the presence of mycobacterium species is confirmed with a high degree of sensitivity.

The basic set of 18 MRM pairs is enough to confirm the presence of mycobacteria from the group listed above with improved reliability and sensitivity, whereas using, in addition, the supplementary set of 34 MRM pairs of Table 2 further provides comprehensive identification of the detected mycobacteria - on a genus level.

The supplementary set of MRM pairs provides supplementary profiles of MAs. The supplementary profiles provide additional, more specific, information concerning the presence of given mycolic acid of the given mycobacteria. In other words, the use of a basic set of MRM pairs indicates the group of mycobacteria present in the sample, whereas the use of the supplementary set of MRM pairs indicates mycobacteria species, on a genus level. Further, some of the MA profiles provide the diagnosis of the other mycobacteria groups.

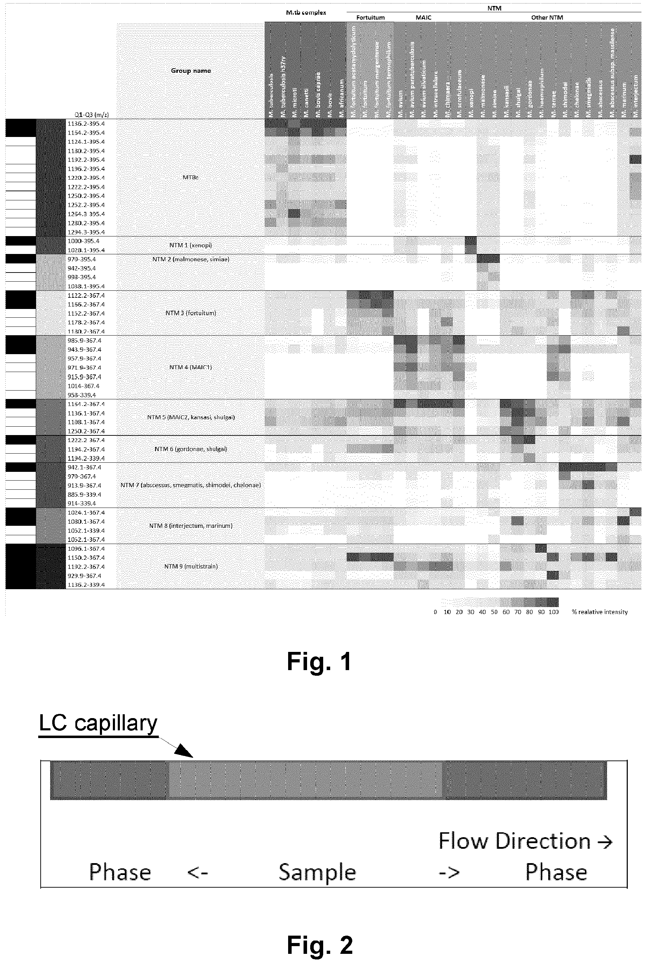

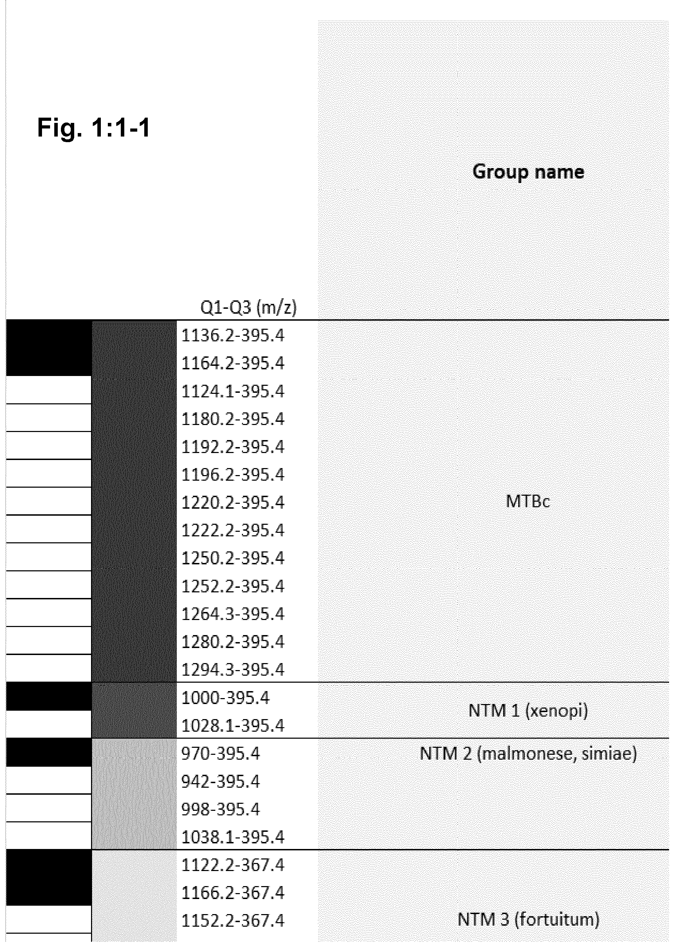

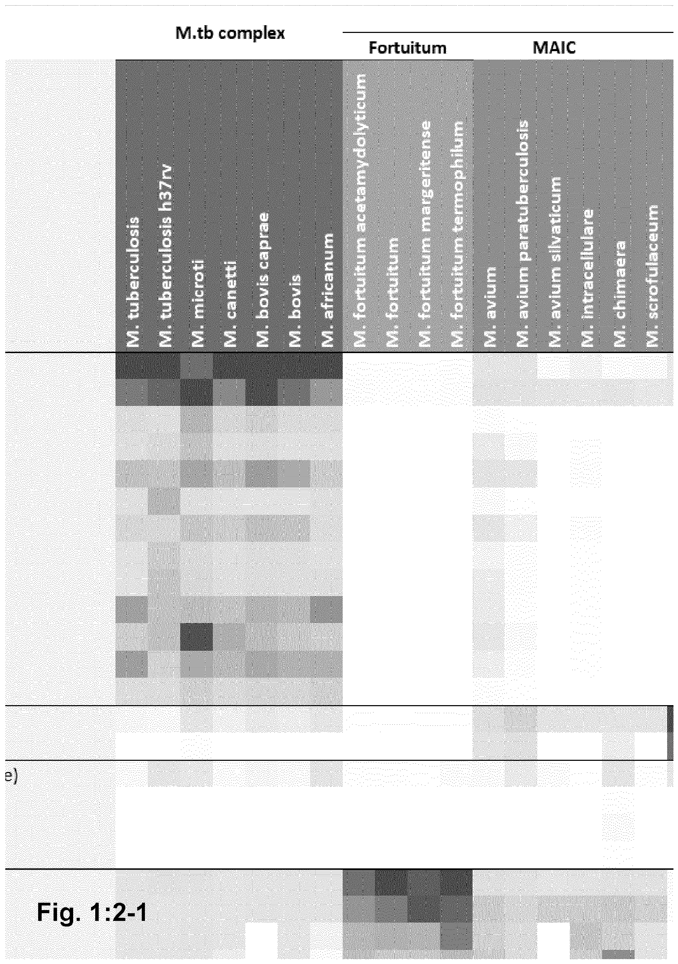

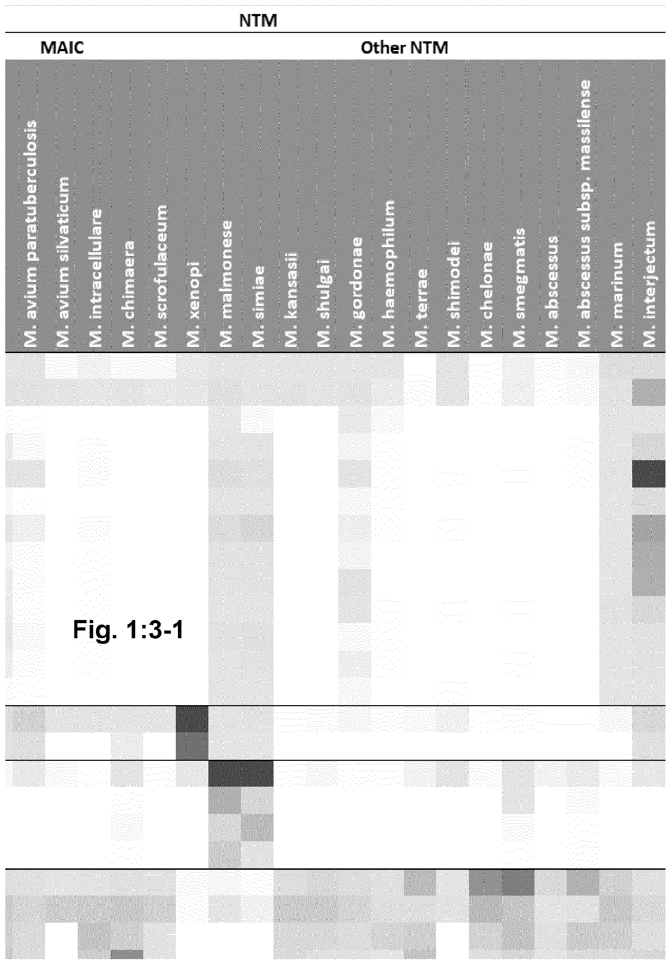

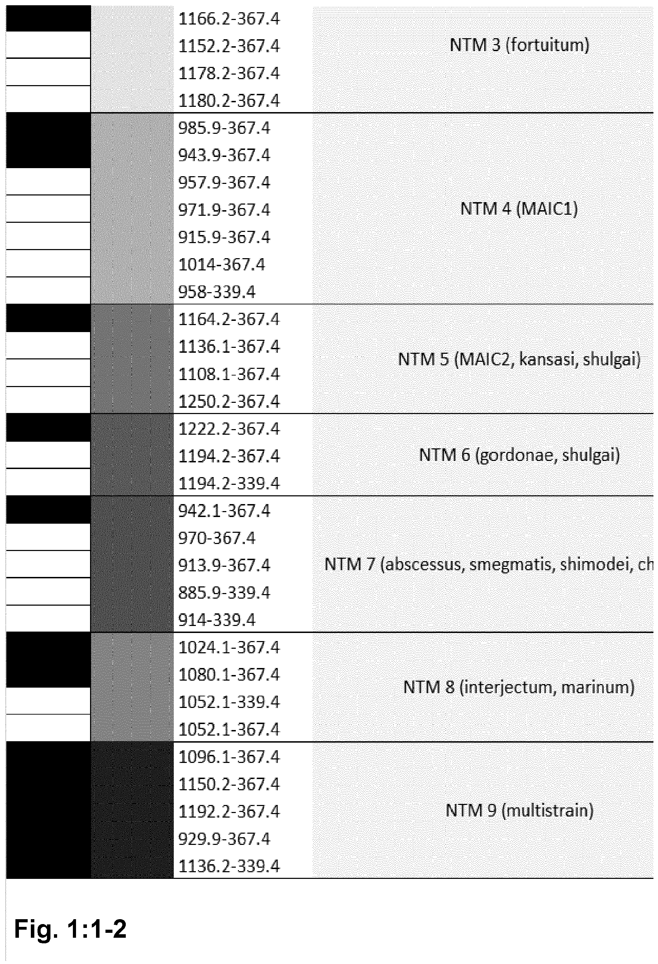

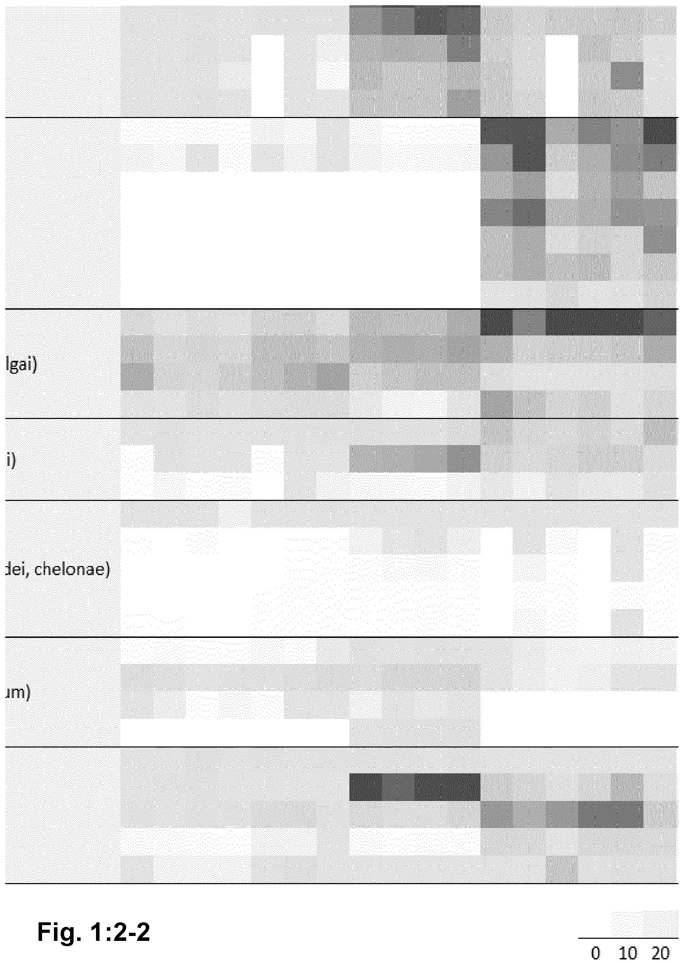

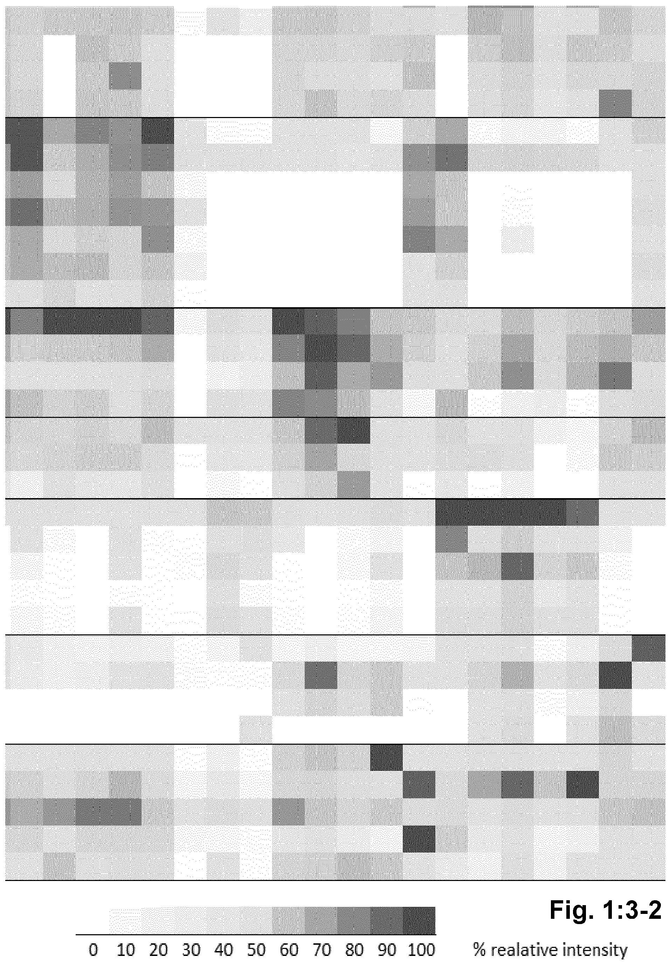

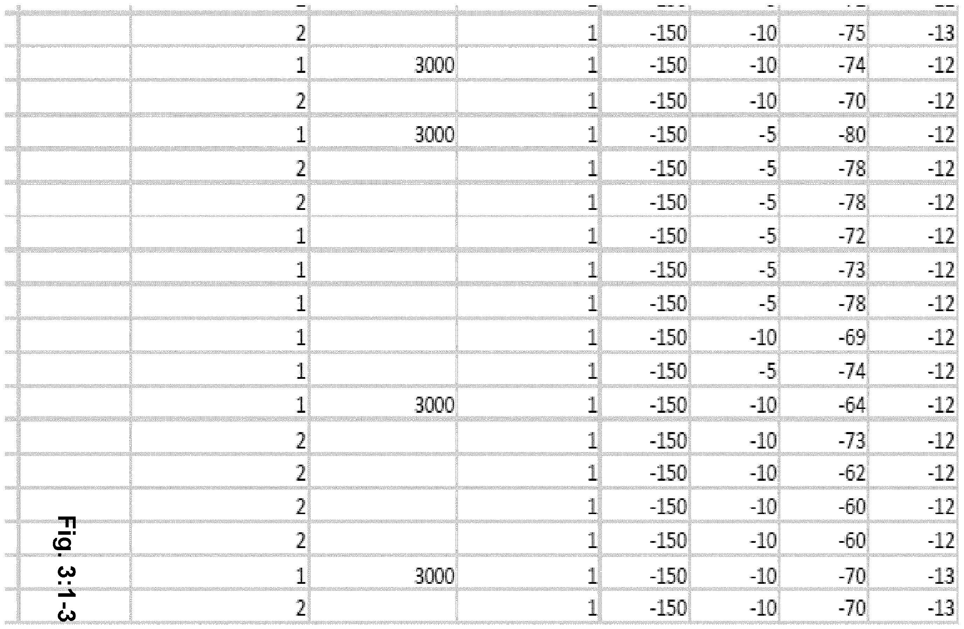

Fig. 1 is a graphic representation of the intensities of the peaks of MS/MS spectra constituting visual representation of the reference profiles of MAs obtained for the basic set of MRM pairs and the supplementary set of MRM pairs - juxtaposed. The basic MRM pairs are marked by black rectangles in the first column on the left, whereas supplementary MRM pairs are marked by white rectangles in the same column. In Fig. 1, the intensities of the peaks from Table 1 and Table 2, are represented graphically in the greyscale of colors, the darker the color the greater the peak intensity. As can be seen in Fig. 1, the basic set of 18 MRM pairs provides detection of the given group of mycobacteria within: MTBc, NTM 1 (xenopi), NTM 2 (malmonese, simiae), NTM 3 (fortuitum), NTM 4 (MAIC1), NTM 4 (MAIC1), NTM 5 (MAIC2, kansasi, shulgai), NTM 6 (gordonae, shulgai), NTM 7 (abscessus, smegmatis, shimodei, chelonae), NTM 8 (interjectum, marinum) and NTM 9 (multistrain), whereas the supplementary set of 34 MRM pairs provides the additional information of the presence of the given mycobacteria species, on a genus level, including: *M. tuberculosis, M. tuberculosis h37rv, M. microti, M. canetti, M. bovis caprae, M. bovis, M. africanum, M. fortuitum acetamydolyticum, M. fortuitum, M. fortuitum margeritense, M. fortuitum termophilum, M. avium, M. avium paratuberculosis, M. avium silvaticum, M. intracellulare, M. chimaera, M. scrofulaceum, M. xenopi, M. malmonese, M. simiae, M. kansasii, M. shulgai, M. gordonae, M. haemophilum, M. terrae, M. shimodei, M. chelonae, M. smegmatis, M*. *abscesses, M*. *abscessus subsp. massilense, M. marinum and M. interjectum.* Thus the additional use of the supplementary set MRM pair can provide more comprehensive results.

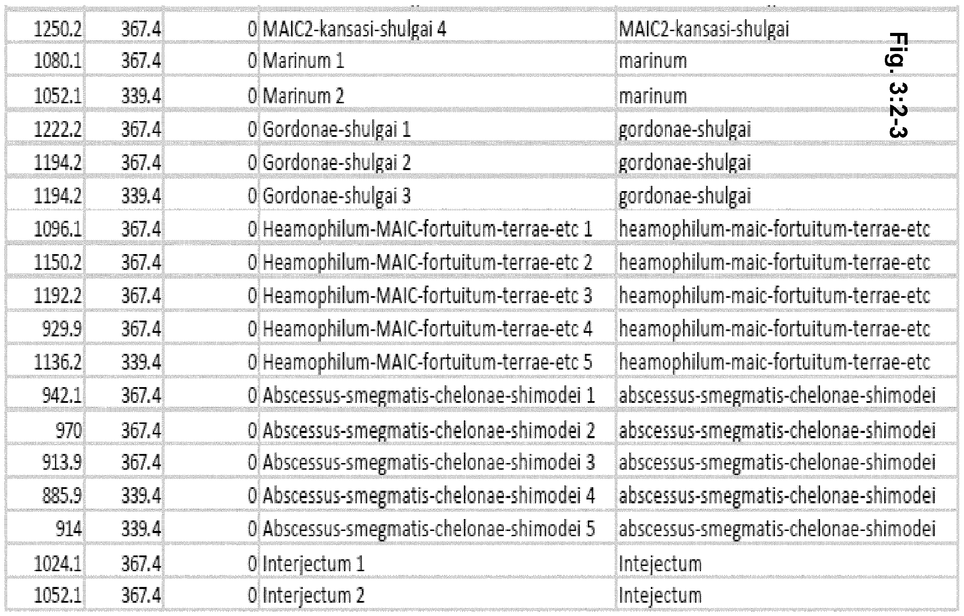

The LC method as used herein does not use the chromatographic separation: Flow Injection Analysis (FIA). Instead, an efficient ionization is provided during the time of flow of investigated liquid sample through a capillary. The investigated sample introduced into the LC analyzer is preferably diluted in a mixture of chloroform and methanol mixed in the volume ratio chloroform: methanol of 50:50 with the addition of 2mM ammonium fluoride (AF). The test sample diluted in the above mixture does not mix with the LC phase - as schematically shown in Fig. 2. The applied gradient flow results in the collection up to several dozen of scans, during the conducted analysis of a single sample. The preferred diameter of the LC capillary is of 0.25 µm or less.

The investigated sample according to the present invention may be for example in a form of sputum or body fluid such as urine, bronchial lavage, bronchoaspirates, etc. notwithstanding their identification. The identification may be additionally taken into account to minimize the risk of contamination between the individual samples. The risk may be minimized by intentionally separating the samples into groups, e.g.: sputum taken from patients that suffer from for lung diseases other than tuberculosis, sputum taken from patients with low level of mycobacteria cells, and sputum taken form patient with an active form of tuberculosis.

### EMBODIMENT 1 - sample preparation and carrying out LC-MS/MS analysis:

The sample of sputum was developed by the alkaline method with the use of N-acetylcysteine (N-AcCys), the method comprising visual assessment the volume of the sputum sample in a vial, and next addition, into the sputum sample, a volume of the solution of 4% sodium hydroxide with 1% N-AcCys that is equal to the volume of the sputum sample (volume ratio of the solution : sputum sample is 1:1). The obtained mixture was incubated at 37°C for 30 minutes. Next, the solution of 2.2% sodium citrate was added to the mixture, in the volume ratio of sodium citrate: sputum starting volume of 1:2. Next 0.067 mol/L of phosphate buffer of pH = 6.8 was added to the mixture, in the volume ration of buffer: sputum starting volume of 1: 4. Next, the mixture was centrifuged at 300 rpm for 30 min. In the case of sample materials other than the sputum, only centrifugation is required to prepare the sample for the LC-MS/MS analysis, whereas, in the case of the development the sputum sample only to LC-MS/MS analysis the addition of sodium citrate and the buffer may be omitted.

After the development, the sample had a form of suspension which was depleted into several portions. Each portion before further treatment was vortexed.

Next, the vortexed portion of the developed sample of volume of minimum 2 mL, was transferred into a separate pyrex vial to prepare the sample for LC-MS/MS analysis. Another portion of the developed and vortexed sample may be additionally taken to conduct bacterioscopy and inoculation analysis, as an option.

Next, the homogenous portion of the developed and vortexed sample (2 mL) was transferred into a vial and tightly closed with a PTFE sealing. Next, the sample was centrifuged at 4500 rpm for 30 min. Subsequently, the supernatant (1.5 mL) was removed, and the obtained sediment 2mL of a solution of 25% potassium hydroxide in methanol (25%KOH in MeOH) was added. The sediment was then vortex-mixed at 3000 rpm for 30 seconds. Next, the vial was tightly sealed with a plug and incubated at 90°C for 60 min. Next, the vial was cooled to room temperature and opened.

The following steps were carried out under a fume hood. The sample was neutralized by the addition of 1.5mL of diluted-in-water hydrochloric acid (18,5% HCl), followed by precipitation of KCI white powder. Next, the sample was subjected to extraction of mycolic acids by addition on the vial 2mL of pure chloroform (ChCl₃) followed by extraction in a liquid-liquid phase, which was carried out by mixing the mixture in a rotator mixer for 30 min. Next, the lower transparent chloroform phase was carefully collected of a volume of a minimum 1 mL. Next, the collected phase was transferred into a glass vial. The vial was closed with a plug and septa. The thus prepared sample was stored in freezing, at temperature -80°C. Alternatively, the sample may be immediately prepared for the analysis.

After de-freezing the probe was mixed with a solution of 4 mM ammonium fluoride in methanol in the volume ratio - sample: solution of 1: 1. Next, the prepared sample was subjected to LC-MS/MS analysis.

### The procedure of LC-MS/MS analysis:

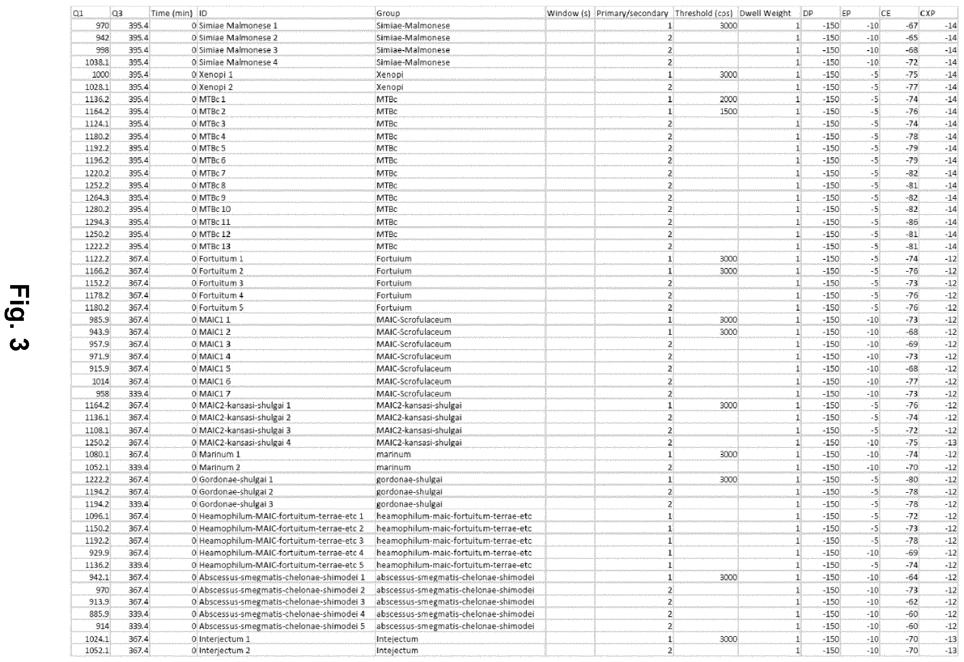

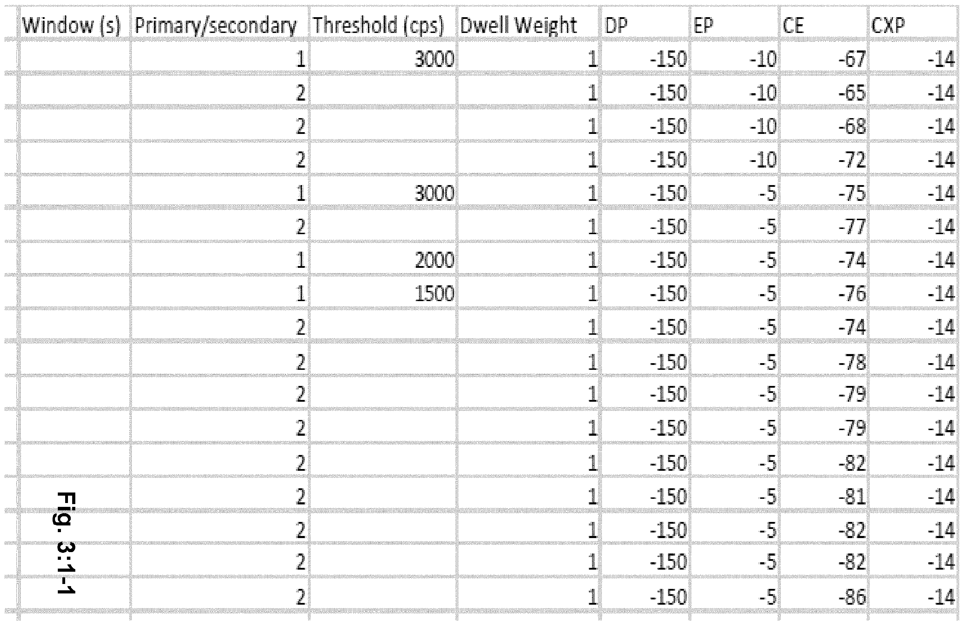

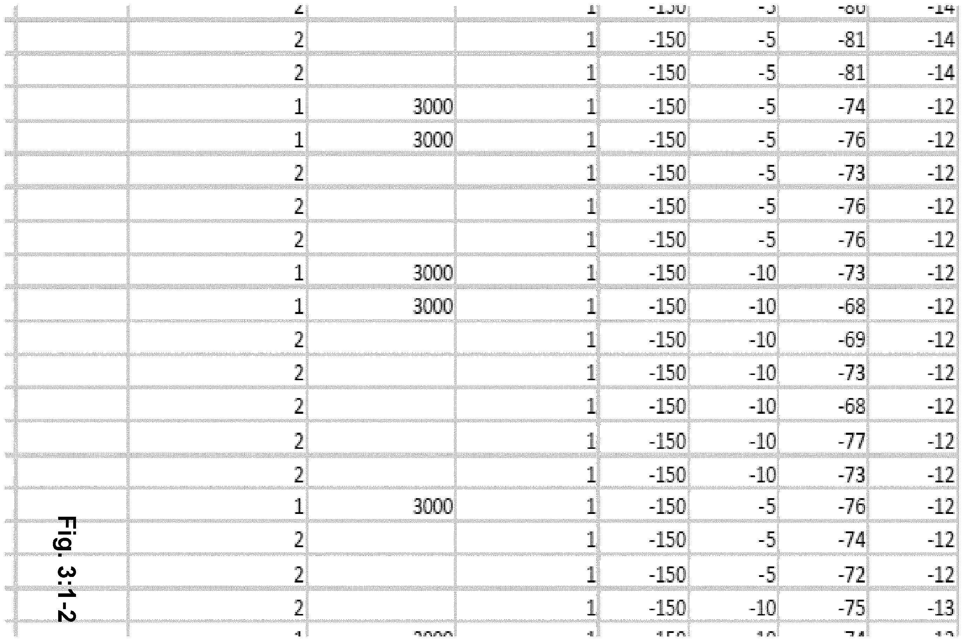

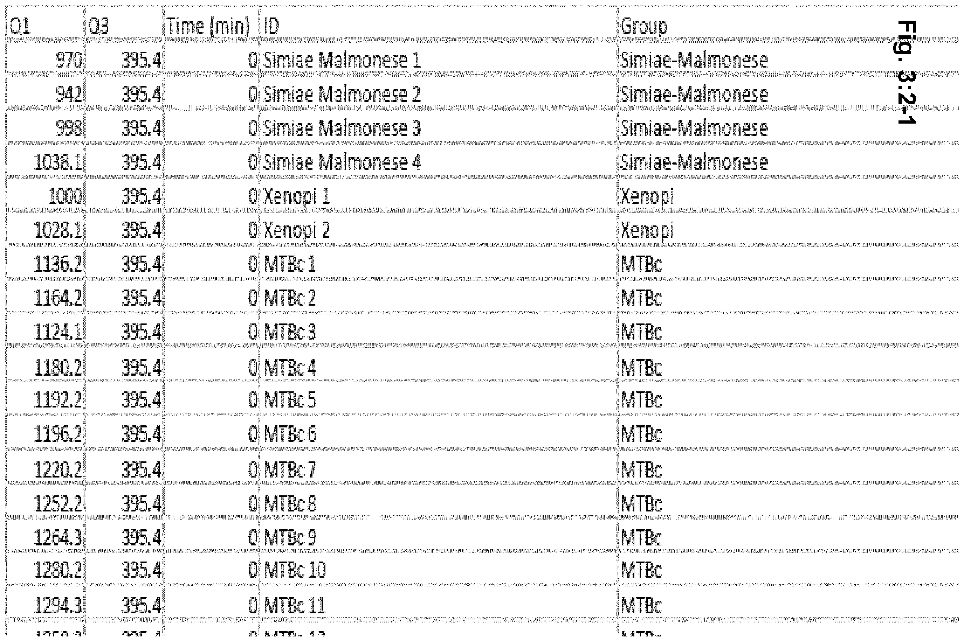

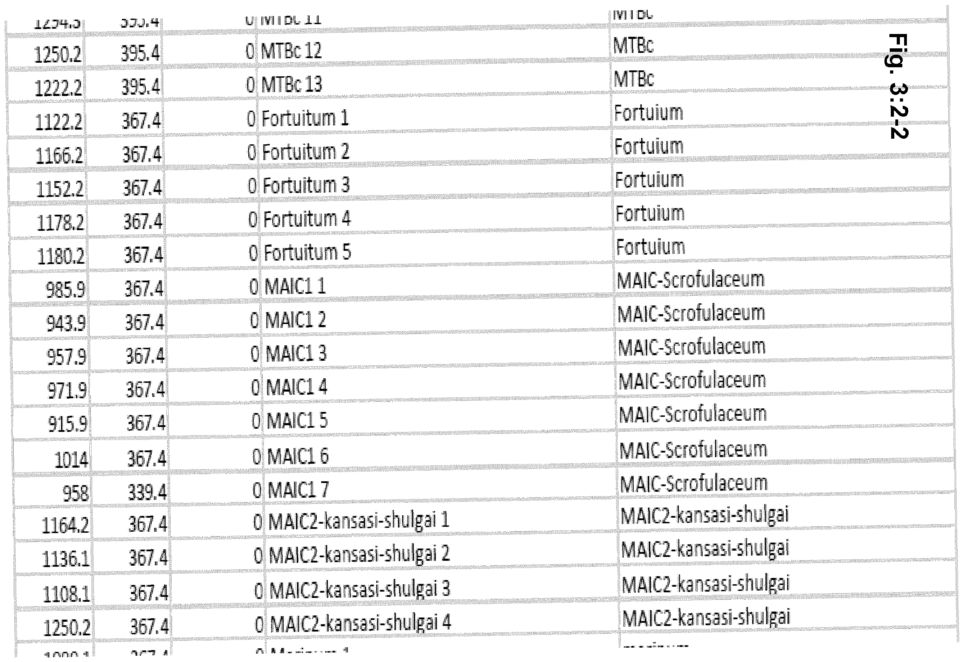

The MRM scanning of the sample was carried out in the mode of negative ionization, with the use of total 52 MRM pairs from Table 1 and Table 2. The signals of the basic set of 18 MRM pairs (Table 1) were applied (triggered) until the threshold intensity was achieved, the threshold values used are specified in Fig. 3 - column 'treshold'. Then, the signals of the supplementary set of 34 MRM pairs (Table 2) was applied (triggered).

### Applied parameters of LC:

Phase A - methanol
Phase B - chloroform
Cleaning phase - chloroform: methanol in the volume ratio of of 80:20
Calibration time: - 0.20 minutes
Injection volume: 50.00 µL

### Parameters of binary gradient applied:

| Time [minutes] | Flow [mL/min] | Ratio of B phase [%] |
|---|---|---|
| 0.00 | 0.2500 | 60.0 |
| 0.39 | 0.2500 | 60.0 |
| 0.40 | 1.0000 | 100.0 |
| 0.90 | 1.0000 | 100.0 |
| 1.00 | 1.0000 | 60.0 |

Autosampler: sampling speed: 10 µL/s; temperature: 8 °C
Rinsing with the washing solution: mode: before and after sampling, time: 10s, speed: 35 µL/s, volume: 500 µL;
The temperature of the chromatography column (only capillary): 30°C.

The sample was subjected to flow through the capillary of a diameter of 0.25 µm, the sample was diluted in the mixture of chloroform: methanol of 50:50 with 2mM of AF, and thus the sample was not merging with the liquid phase.

### Applied parameters of MS/MS:

| | |
|---|---|
| Scanninge: | MRM |
| Scheduled MRM: | Yes (advanced) |
| Polarization: | unegative |
| Desired scanning time: | 0.5000 sec |
| Resolution Q1: | Low |
| Resolution Q3: | Unit |
| MRM Pause: | 3.0000 msec |

Ion source parameters: CUR: 20.00 psi, TEM: 450.00 °C, GS1: 50.00 psi, GS2: 70.00 psi, CAD: Medium, IS: -4500.00 V.

The MRM pairs and the scanning parameters were set up in Fig. 3. in the table.

The obtained results were next compared with the reference data, in the following steps: the computer cooperating with the LC-MS/MS analyzer uses the algorithm which, based on a required pattern and provides a histogram of intensities of respective MRM transitions, comprising the histogram reference transitions which is already available for the algorithm. The same is provided for the investigated sample. If one or more than one of the defined MRM transitions are not obtained as result data - for the scanned (investigated) sample, such MRM transition is added as "0" value. The algorithm counts the area of the histogram for the pattern (Sref), whereas each MRM transition is provided as a rectangle of sides: base = 1, height = value of intensity. Next, the algorithm creates a third histogram being the non-compliance area constituting the difference between the sample data and the reference data, next the algorithm counts the area of the third histogram in the same manner (Sdiff). The absolute values are taken from the difference values of the MRM transition. The compatibility for the pattern is calculated using the formula: 100*(1-(Sdiff-Sref)), wherein the negative values are returned as "0". The compatibility for the tested/unknown strain of bacteria is treated as the highest match of the patterns that belong to the reference strain.

### EMBODIMENT 2 - comparison of the obtained results with the reference data:

a) the pattern P with MRM transitions of the following mycolic acids: KM01, KM02, KM03, KM04, KM05 was obtained for the reference sample. The pattern intensities for the respective mycolic acids were as follows: 100, 20, 40, 60, 10. The area of the histogram for the pattern was calculated: Sref = 100+20+40+60+10 = 230. As a result of the LC-MS/MS analysis of the investigated sample A, the following MRM transitions data were obtained: KM01, KM03, KM05 of the respective values of 20, 30, 50. Thus the pattern of sample A was replenished with KM02, KM04, thus obtaining values of intensities of 20,0,30,0,50. Sdiff = |100-20| + |20-0| + |40-30| + |60-0| + |10-50| = 210. The compatibility of this two patterns (P, A) = 100*(1-210/230) = 8.7%. The obtained result means that the correlation is low - the identification certainty is weak.
b) the sample B was found to comprise the pattern (B): KM01, KM02, KM03, KM04, KM05, 100,19,45,55,10. Sdiff = |100-100| + |20-19| + |40-45| + |60-55| + |10-10| = 11. Compatibility (P,B) = 100*(1-11/230) = 95.2%. The obtained result means that the correlation is high - the identification certainty is strong.
c) the sample C was found to comprise the pattern (C ): KM01, KM02, KM03, KM04, KM05, 100,100,100,100,100. Sdiff = 270. Compatibility (P,C) = 100*(1-270/230) = -17.4%; the result was returned as 0%. The obtained result means that there is no correlation - no identification: no mycobacteria in the sample detected.

## Claims

1. A method for detecting a presence of *mycobacterium* species in a test sample by subjecting the test sample to a liquid chromatographic-tandem mass spectrometry (LC-MS/MS) analysis using a multiple reaction monitoring (MRM) scanning mode with an LC-MS/MS analyzer comprising a liquid chromatographic (LC) instrument for generating precursor ions and a triple quadrupole (Q1, q, Q3) instrument for transiting the precursor ions into fragment ions, **characterized by** comprising the steps of:
- scanning the precursor ions of a predetermined value of mass-to-charge ratio (m/z) with a first quadrupole (Q1) and scanning the fragment ions of a predetermined value of mass-to-charge ratio (m/z) with a third quadrupole (Q3), wherein the scanning is performed for a set of pairs of ions, each of the pairs consisting of one precursor ion and one fragment ion of said precursor, the pairs of ions comprising the basic set of pairs of ions of the following m/z values: 1136.2-395.4, 1164.2-395.4, 1000-395.4, 970-395.4, 1122.2-367.4, 1166.2-367.4, 985.9-367.4, 943.9-367.4, 1164.2-367.4, 1222.2-367.4, 942.1-367.4, 1024.1-367.4, 1080.1-367.4, 1096.1-367.4, 1150.2-367.4, 1192.2-367.4, 929.9-367.4, 1136.2-339.4;
- based on data received from the scanning, obtaining profiles of mycolic acids (MAs), if present in the test sample,
- comparing the obtained profiles of mycolic acids with reference profiles of mycolic acids obtained by subjecting the reference sample to a liquid chromatographic-tandem mass spectrometry (LC-MS/MS) analysis using a multiple reaction monitoring (MRM) scanning mode, to access compatibility of the sample profiles of mycolic acids and the reference profiles of mycolic acids, and
- based on the assessed compatibility, identifying whether *mycobacterium* species is present in the test sample and if so, classifying the *mycobacteria* species into one or more groups of the following classification scheme:
o a group belonging to *M*. *tuberculosis* complex (MTBc), comprising: *M*. *tuberculosis, M. tuberculosis h37rv, M. microti, M. canetti, M. bovis caprae, M. bovis, M. africanum, M. interjectum,*
o a first group not belonging to *M*. *tuberculosis* complex (NTM 1), comprising: *M*. xenopi,
o a second group not belonging to *M*. *tuberculosis* complex (NTM 2), comprising: *M*. *malmonese, M. simiae,*
o a third group not belonging to *M*. *tuberculosis* complex (NTM 3), comprising: *M. fortuitum acetamydolyticum, M. fortuitum, M. fortuitum margeritense, M. fortuitum termophilum, M. chimaera, M. chelonae, M. smegmatis, M. marinum,*
o a fourth group not belonging to *M*. *tuberculosis* complex (NTM 4) comprising: *M. avium, M. avium paratuberculosis, M. avium silvaticum, M. intracellulare, M. chimaera, M. scrofulaceum, M. terrae, M. shimodei,*
o a fifth group not belonging to *M*. *tuberculosis* complex (NTM 5) comprising: *M. avium, M. avium paratuberculosis, M. avium silvaticum, M. intracellulare, M. chimaera, M. scrofulaceum, M. kansasii, M. shulgai, M. terrae,*
o a sixth group not belonging to *M*. *tuberculosis* complex (NTM 6) comprising: *M. gordonae, M. shulgai, M. fortuitum acetamydolyticum, M. fortuitum, M. fortuitum margeritense, M. fortuitum termophilum,*
o a seventh group not belonging to *M*. *tuberculosis* complex (NTM 7) comprising: *M. shimodei, M. chelonae, M. smegmatis, M*. *abscessus, M*. *abscessus subsp. Massilense,*
o an eighth group not belonging to *M*. *tuberculosis* complex (NTM 8) comprising: *M. marinum, M. interjectum, M. shulgai,* and
o a ninth group not belonging to *M*. *tuberculosis* complex (NTM 9) comprising: *M. fortuitum acetamydolyticum, M. fortuitum, M. fortuitum margeritense, M. fortuitum termophilum, M. avium, M. avium paratuberculosis, M. avium silvaticum, M. intracellulare, M. chimaera, M. scrofulaceum, M. kansasii, M. haemophilum, M. terrae, M. chelonae, M. smegmatis, M*. *abscessus, M*. *abscessus subsp. Massilense.*

2. The method according to claim 1 wherein the ionization is performed in the negative mode.

## Patentansprüche

1. Verfahren zum Nachweisen eines Vorhandenseins von *Mykobakterien-Spezies* in einer Testprobe, indem die Testprobe einer Flüssigkeitschromatografie-Tandem-Massenspektrometrie(LC-MS/MS)-Analyse unter Verwendung eines Multiple-Reaction-Monitoring-Scanmodus (MRM) mit einem LC-MS/MS-Analysator unterzogen wird, der ein Flüssigkeitschromatografiegerät (LC) zum Erzeugen von Vorläuferionen und ein Dreifach-Quadrupol-Gerät (Q1, q, Q3) zum Umwandeln der Vorläuferionen in Fragmentionen umfasst, **dadurch gekennzeichnet, dass** es die folgenden Schritte umfasst:
- Scannen der Vorläuferionen eines vorgegebenen Wertes des Masse-zu-Ladung-Verhältnisses (m/z) mit einem ersten Quadrupol (Q1) und Scannen der Fragmentionen eines vorgegebenen Wertes des Masse-zu-Ladung-Verhältnisses (m/z) mit einem dritten Quadrupol (Q3), wobei das Scannen für eine Reihe von lonenpaaren durchgeführt wird, wobei jedes der Paare aus einem Vorläuferion und einem Fragmention des Vorläufers besteht, wobei die Ionenpaare die Basisreihe von lonenpaaren der folgenden m/z-Werte umfassen: 1136,2-395,4, 1164,2-395,4, 1000-395,4, 970-395,4, 1122,2-367,4, 1166,2-367,4, 985,9-367,4, 943,9-367,4, 1164,2-367,4, 1222,2-367,4, 942,1-367,4, 1024,1-367,4, 1080,1-367,4, 1096,1-367,4, 1150,2-367,4, 1192,2-367,4, 929,9-367,4, 1136,2-339,4;
- Erhalten von Profilen der Mykolsäuren (MAs), sofern in der Testprobe vorhanden, auf der Grundlage der durch das Scannen empfangenen Daten,
- Vergleichen der erhaltenen Mykolsäureprofile mit Referenzprofilen von Mykolsäuren, die durch Unterziehen der Referenzprobe einer Flüssigchromatografie-Tandem-Massenspektrometrie(LC-MS/MS)-Analyse unter Verwendung eines Multiple-Reaction-Monitoring-Scanmodus (MRM) erhalten wurden, um Zugriff auf Kompatibilität der Probenprofile von Mykolsäuren und der Referenzprofile von Mykolsäuren zu erlangen und
- auf Grundlage der bewerteten Kompatibilität Feststellen, ob in der Testprobe eine *Mykobakterien-Spezies* vorhanden ist, und wenn ja, Klassifizieren der *Mykobakterien-*Spezies in eine oder mehrere Gruppen des folgenden Klassifizierungsschemas:
o eine dem *M. tuberculosis-Komplex* (MTBc) zugehörige Gruppe, umfassend: *M. tuberculosis, M. tuberculosis h37rv, M. microti, M. canetti, M. bovis caprae, M. bovis, M. africanum, M. interjectum,*
o eine erste Gruppe, die nicht zum *M. tuberculosis-Komplex* (NTM 1) gehört, umfassend: M. xenopi,
o eine zweite Gruppe, die nicht zum *M. tuberculosis-Komplex* (NTM 2) gehört, umfassend: *M. malmonese, M. simiae,*
o eine dritte Gruppe, die nicht zum *M. tuberculosis-Komplex* (NTM 3) gehört, umfassend: *M. fortuitum acetamydolyticum, M. fortuitum, M. fortuitum margeritense, M. fortuitum termophilum, M. chimaera, M. chelonae, M. smegmatis, M. marinum,*
o eine vierte Gruppe, die nicht zum *M. tuberculosis-Komplex* (NTM 4) gehört, umfassend: *M. avium, M. avium paratuberculosis, M. avium silvaticum, M. intrazellulare, M. chimaera, M. scrofulaceum, M. terrae, M. shimodei,*
o eine fünfte Gruppe, die nicht zum *M. tuberculosis-Komplex* (NTM 5) gehört, umfassend: *M. avium, M. avium paratuberculosis, M. avium silvaticum, M. intrazellulare, M. chimaera, M. scrofulaceum, M. kansasii, M. shulgai, M. terrae,*
o eine sechste Gruppe, die nicht zum *M. tuberculosis-Komplex* (NTM 6) gehört, umfassend: *M. gordonae, M. shulgai, M. fortuitum acetamydolyticum, M. fortuitum, M. fortuitum margeritense, M. fortuitum termophilum,*
o eine siebte Gruppe, die nicht zum *M. tuberculosis-Komplex* (NTM 7) gehört, umfassend: *M. shimodei, M. chelonae, M. smegmatis, M. abscessus, M. abscessus subsp. Massilense,*
o eine achte Gruppe, die nicht zum *M. tuberculosis-Komplex* (NTM 8) gehört, umfassend: *M. marinum, M. interjectum, M. shulgai* und
o eine neunte Gruppe, die nicht zum *M. tuberculosis-Komplex* (NTM 9) gehört, umfassend: *M. fortuitum acetamydolyticum, M. fortuitum, M. fortuitum margeritense, M. fortuitum termophilum, M. avium, M. avium paratuberculosis, M. avium silvaticum, M. intrazellulare, M. chimaera, M. scrofulaceum, M. kansasii, M. haemophilum, M. terrae, M. chelonae, M. smegmatis, M. abscessus, M. abscessus subsp. Massilense.*

2. Verfahren nach Anspruch 1, wobei die Ionisierung im negativen Modus durchgeführt wird.

## Revendications

1. Procédé permettant la détection de la présence d'espèces de *mycobactéries* dans un échantillon d'essai en soumettant l'échantillon d'essai à une analyse par chromatographie en phase liquide-spectrométrie de masse en tandem (CL-SM/SM) en utilisant un mode de balayage de surveillance de réactions multiples (MRM) avec un analyseur CL-SM/SM comprenant un instrument de chromatographie en phase liquide (CL) permettant la génération d'ions précurseurs et un instrument quadripôle triple (Q1, q, Q3) permettant la transition des ions précurseurs en ions fragments, **caractérisé en ce qu'**il comprend les étapes de :
- balayage des ions précurseurs d'une valeur prédéfinie de rapport masse sur charge (m/z) avec un premier quadripôle (Q1) et balayage des ions fragments d'une valeur prédéfinie de rapport masse sur charge (m/z) avec un troisième quadripôle (Q3), ledit balayage étant effectué pour un ensemble de paires d'ions, chacune des paires étant constituée d'un ion précurseur et d'un ion fragment dudit précurseur, les paires d'ions comprenant l'ensemble de base des paires d'ions des valeurs m/z suivantes : 1136,2 à 395,4, 1164,2 à 395,4, 1000 à 395,4, 970 à 395,4, 1122,2 à 367,4, 1166,2 à 367,4, 985,9 à 367,4, 943,9 à 367,4, 1164,2 à 367,4, 1222,2 à 367,4, 942,1 à 367,4, 1024,1 à 367,4, 1080,1 à 367,4, 1096,1 à 367,4, 1150,2 à 367,4, 1192,2 à 367,4, 929,9 à 367,4, 1136,2 à 339,4 ;
- sur la base des données reçues du balayage, obtention des profils d'acides mycoliques (AM), s'ils sont présents dans l'échantillon d'essai,
- comparaison des profils obtenus d'acides mycoliques à des profils de référence d'acides mycoliques obtenus en soumettant l'échantillon de référence à une analyse par chromatographie en phase liquide-spectrométrie de masse en tandem (CL-SM/SM) en utilisant un mode de balayage de surveillance de réactions multiples (MRM), pour accéder à la compatibilité des profils d'échantillons d'acides mycoliques et des profils de référence d'acides mycoliques, et
- sur la base de la compatibilité évaluée, identification pour savoir si une espèce de *mycobactérie* est présente dans l'échantillon d'essai et, le cas échéant, classement de l'espèce de *mycobactérie* dans un ou plusieurs groupes du schéma de classification suivant :
o un groupe appartenant au complexe de *M. tuberculosis* (MTBc), comprenant : *M. tuberculosis, M. tuberculosis h37rv, M. microti, M. canetti, M. bovis caprae, M. bovis, M. africanum, M. interjectum,*
o un premier groupe n'appartenant pas au complexe de *M. tuberculosis* (NTM 1), comprenant : *M. xenopi,*
o un deuxième groupe n'appartenant pas au complexe de *M. tuberculosis* (NTM 2), comprenant : *M. malmonese, M. simiae,*
o un troisième groupe n'appartenant pas au complexe de *M. tuberculosis* (NTM 3), comprenant : *M. fortuitum acetamydolyticum, M. fortuitum, M. fortuitum margeritense, M. fortuitum termophilum, M. chimaera, M. chelonae, M. smegmatis, M. marinum,*
o un quatrième groupe n'appartenant pas au complexe de *M. tuberculosis* (NTM 4) comprenant : *M. avium, M. avium paratuberculosis, M. avium silvaticum, M. intracellulare, M. chimaera, M. scrofulaceum, M. terrae, M. shimodei,*
o un cinquième groupe n'appartenant pas au complexe de *M. tuberculosis* (NTM 5) comprenant : *M. avium, M. avium paratuberculosis, M. avium silvaticum, M. intracellulare, M. chimaera, M. scrofulaceum, M. kansasii, M. shulgai, M. terrae,*
o un sixième groupe n'appartenant pas au complexe de *M. tuberculosis* (NTM 6) comprenant : *M. gordonae, M. shulgai, M. fortuitum acetamydolyticum, M. fortuitum, M. fortuitum margeritense, M. fortuitum termophilum,*
o un septième groupe n'appartenant pas au complexe de M. *tuberculosis* (NTM 7) comprenant : *M. shimodei, M. chelonae, M. smegmatis, M. abscessus, M. abscessus subsp. Massilense,*
o un huitième groupe n'appartenant pas au complexe de *M. tuberculosis* (NTM 8) comprenant : *M. marinum, M. interjectum, M. shulgai* et
o un neuvième groupe n'appartenant pas au complexe de *M. tuberculosis* (NTM 9) comprenant : *M. fortuitum acetamydolyticum, M. fortuitum, M. fortuitum margeritense, M. fortuitum termophilum, M. avium, M. avium paratuberculosis, M. avium silvaticum, M. intracellulare, M. chimaera, M. scrofulaceum, M. kansasii, M. haemophilum, M. terrae, M. chelonae, M. smegmatis, M. abscessus, M. abscessus subsp. Massilense.*

2. Procédé selon la revendication 1, l'ionisation étant effectuée en mode négatif.
